(19) **Europäisches Patentamt European Patent Office Office européen des brevets**

(11) **EP 4 061 168 B1**

(12) **FASCICULE DE BREVET EUROPEEN**

(45) Date de publication et mention de la délivrance du brevet:
**22.01.2025 Bulletin 2025/04**

(21) Numéro de dépôt: **20804557.5**

(22) Date de dépôt: **16.11.2020**

(51) Classification Internationale des Brevets (IPC):
*A41D 31/18* (2019.01)   *A61F 13/06* (2006.01)
*A41D 31/00* (2019.01)   *A41D 13/12* (2006.01)
*A41D 1/08* (2018.01)   *A41B 9/00* (2006.01)
*A41B 11/02* (2006.01)   *A61F 13/00* (2024.01)
*A41B 9/04* (2006.01)   *D04B 1/24* (2006.01)
*A61F 13/08* (2006.01)   *A61F 5/30* (2006.01)
*D04B 1/26* (2006.01)   *A41D 13/00* (2006.01)

(52) Classification Coopérative des Brevets (CPC):
**A41B 9/04; A41B 9/004; A41B 11/02; A41D 1/088;
A41D 13/1254; A41D 31/0005; A41D 31/18;
A61F 5/30; D04B 1/243;** A41D 13/0015;
A61F 13/08; D10B 2509/028

(86) Numéro de dépôt international:
**PCT/EP2020/082222**

(87) Numéro de publication internationale:
**WO 2021/099251 (27.05.2021 Gazette 2021/21)**

(54) **VÊTEMENT DE COMPRESSION ET/OU DE CONTENTION POUR LE TRAITEMENT DU LYMPHOEDÈME**

KOMPRESSIONS- UND/ODER KONTRAKTIONSKLEIDUNG ZUR LYMPHÖDEMBEHANDLUNG

COMPRESSION AND/OR CONTENTION GARMENT FOR LYMPHOEDEMA TREATMENT

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **18.11.2019 FR 1912843**

(43) Date de publication de la demande:
**28.09.2022 Bulletin 2022/39**

(73) Titulaire: **Thuasne**
**92300 Levallois Perret (FR)**

(72) Inventeurs:
• **AGUD, Adrien**
**07610 Lemps (FR)**
• **GALLIEN, Nathalie**
**42100 Saint-Etienne (FR)**
• **RICHARD, Laurence**
**43240 Saint Just Malmont (FR)**
• **JOUMARD, Leslie**
**42100 Saint-Etienne (FR)**
• **FAVIER, Angélique**
**42170 Saint Just Saint Rambert (FR)**

(74) Mandataire: **Lavoix**
**2, place d'Estienne d'Orves**
**75441 Paris Cedex 09 (FR)**

(56) Documents cités:
EP-A2- 2 449 901    EP-B1- 2 449 901
WO-A1-2011/124849    FR-A1- 3 027 195
US-A1- 2015 173 428    US-A1- 2016 338 417
US-A1- 2018 303 179

EP 4 061 168 B1

**Description**

La présente invention concerne un vêtement de compression et/ou de contention pour le traitement du lymphoedème, le vêtement comprenant un corps principal, le corps principal étant réalisé dans un textile de compression.

Des vêtements de compression pour le traitement d'un lymphoedème sont déjà connus.

Par exemple, le modèle de short de compression Compreshorts ® de SIGVARIS ® est notamment indiqué pour le traitement d'un lymphoedème génital et présente, d'après le fournisseur, une classe de compression comprise entre 10 à 15 mmHg.

Cependant, des professionnels de santé et des patients reproche un manque d'efficacité des produits disponible sur le marché en raison d'un manque de force et de maintien sur les zones principales de localisation de l'oedème, comme les parties génitales, le pubis et/ou les zones inguinales.

Le document US 2018/303179 A1 décrit un vêtement de compression formé d'un tissu étirable avec une élasticité uniforme. Une portion circonférentielle du vêtement entoure un membre de l'utilisateur et est caractérisée par différent degré d'étirement, mais des forces de compression circonférentielles identiques, lorsqu'elle est portée par l'utilisateur, e long d'une direction axiale.

D'autres vêtements de compression sont connus de EP 2 449 901 A2, US 2015/173428 A1, WO 2011/124849 A1 et FR 3 027 195 A1.

Un but de l'invention est donc de proposer un vêtement de compression et/ou de contention efficace dans le traitement du lymphoedème.

A cet effet, l'invention a pour objet un vêtement de compression et/ou de contention pour le traitement du lymphoedème selon la revendication 1.

La première et la deuxième zone ciblée permettent d'appliquer des efforts différemment pour aider à drainer le membre oedématié en le contenant et en étant apte à s'adapter à la morphologie de la zone à traiter et/ou à appliquer une pression à des endroits précis où celle-ci doit être importante pour drainer efficacement le membre. La première zone ciblée permet notamment d'apporter plus de maintien par une rigidité plus importante qu'en dehors des zones ciblées. La deuxième zone ciblée permet d'appliquer une force importante dès que le volume du membre augmente d'une valeur minimale, par exemple, en cas de gonflement du lymphoedème.

Le vêtement de compression et/ou de contention peut en outre présenter une ou plusieurs caractéristiques des revendications 2 à 10, considérée(s) individuellement ou selon toutes les combinaisons techniquement possibles.

D'autres caractéristiques et avantages de l'invention apparaîtront à la lecture de la description qui suit de modes de réalisation de l'invention, donnée à titre d'exemple uniquement et en référence aux dessins dans lesquels :

- la figure 1 est une vue schématique de face d'un vêtement selon un mode de réalisation de l'invention,
- la figure 2 est une vue schématique de dos du vêtement de la figure 1, et
- les figures 3 et 4 sont des vues schématiques de face et de dos respectivement d'un exemple de patronage du vêtement des figures 1 et 2.

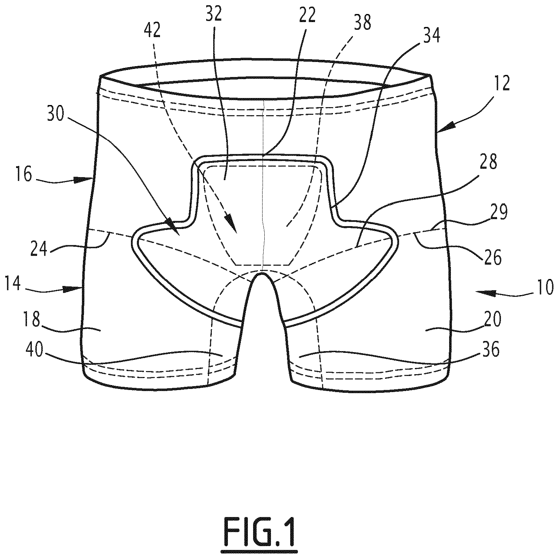

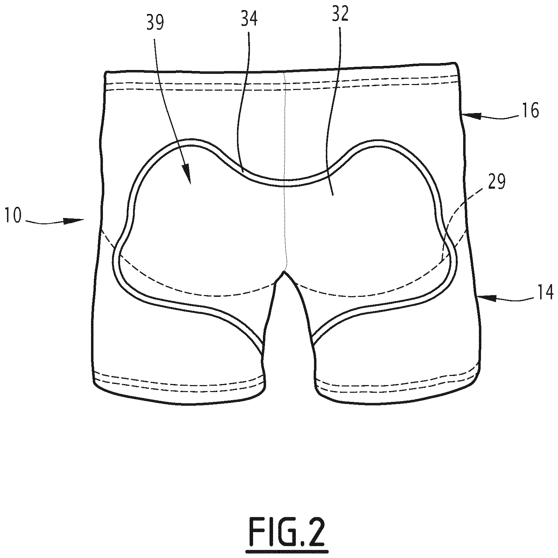

Un mode de réalisation d'un vêtement 10 de compression et/ou contention pour le traitement du lymphoedème selon l'invention est représenté sur les figures 1 et 2.

Le vêtement 10 est ici destiné à traiter le lymphoedème génital et/ou pelvien.

Le vêtement 10 est, dans l'exemple représenté, un panty, c'est-à-dire une culotte à taille haute et descendant sur les cuisses.

En chaque point du vêtement, on définit une direction circonférentielle, prévue pour correspondre à une direction circonférentielle d'un plan transversal d'un utilisateur portant le vêtement.

Le vêtement 10 est adapté à la morphologie d'un utilisateur. Par exemple, le vêtement 10 est développé en différentes tailles, la taille de vêtement adaptée à un utilisateur étant choisie en fonction du tour de bassin de l'utilisateur.

Le vêtement 10 comprend un corps principal 12.

Dans un mode de réalisation, le corps principal 12 s'étend sur toute la surface du vêtement 10.

Le corps principal 12 présente une face interne prévue pour s'étendre au moins partiellement contre l'utilisateur et une face externe opposée à la face interne.

Le corps principal 12 est réalisé dans un textile de compression.

Le corps principal 12 présente ici une unique couche ou épaisseur de textile de compression.

Le textile de compression est ici un tissu bi-élastique, c'est-à-dire élastique selon deux directions d'extension du tissu non parallèle, ici la direction d'extensibilité et la direction perpendiculaire à la direction d'extensibilité.

Alternativement, le textile de compression est un tissu élastique selon une unique direction.

Alternativement, le textile de compression est un tricot bi-élastique.

Le textile de compression présente une élasticité strictement supérieure à 70% dans au moins une direction d'extensibilité.

La direction d'extensibilité du textile de compression correspond sur l'ensemble du corps principal 12 à la direction circonférentielle.

Le textile de compression présente un module selon la direction d'extensibilité.

Le module est mesuré selon la norme NF EN 14704-1 datant de juin 2005 sur la détermination de l'élasticité des étoffes.

Plus particulièrement, une bande du matériau est découpée.

La bande s'étend entre deux extrémités selon une direction d'intérêt dans laquelle le module est à mesurer, ici la direction d'extensibilité.

La bande présente une largeur donnée mesurée perpendiculairement à la direction d'intérêt.

La bande est étirée au moins un nombre donné de cycles d'étirage, le nombre donné est égal à 5.

Pour chaque cycle, la bande est étirée selon la direction d'intérêt par une force augmentant progressivement jusqu'à une force prédéterminée, par exemple égale à 6N par centimètre de largeur de la bande. Par exemple, la bande présente une largeur de 5 centimètres et est donc étirée par une force augmentant progressivement jusqu'à 30N.

La bande est, par exemple, étirée à l'aide d'un dynamomètre, chaque extrémité de la bande étant maintenue par une pince respective, par exemple l'une fixe alors que la seconde réalise les cycles de traction/relaxation par des allers-retours suivant un axe.

L'allongement de la bande en fonction de la force appliquée est mesuré.

Puis, l'étirage de la bande est diminué par un retour progressif à la position initiale, plus particulièrement de la pince en mouvement vers sa position initiale. Un nouveau cycle d'étirage est alors susceptible d'être commencé.

Le module est ici la force correspondant à un allongement de 40% au cours du cinquième cycle pendant la phase d'augmentation progressive de la force.

Le textile de compression présente un module selon la direction d'extensibilité supérieure ou égale à 3,5 N pour une bande de 5 centimètres de large.

En outre, le textile de compression est tel que, en dehors du premier cycle d'étirage, les courbes représentant l'allongement en fonction de la force appliquée au cours des différents cycles se superposent à 5% près, au moins entre les cycles compris entre le deuxième et le cinquième cycles inclus.

Cela permet de réaliser un produit stable dans le temps.

Par ailleurs, lors du premier cycle d'étirage, le textile de compression requiert une force d'étirage selon la direction d'extensibilité supérieure ou égale à 7N pour obtenir un allongement de 40% d'une bande de 5 centimètres de large.

Cela permet notamment d'atteindre une force souhaitée avec un étirage peu important. Ainsi, le vêtement présente avantageusement des dimensions proches de celles de l'utilisateur portant le vêtement, l'enfilage étant alors facilité.

Le textile de compression comprend, par exemple, plus particulièrement est constitué, de polyamide et d'élasthanne. Le textile de compression est ici constitué de 40% à 50%, plus particulièrement 48%, d'élasthanne et de 50% à 60%, plus particulièrement 52%, de polyamide.

Dans le mode de réalisation représenté, le corps principal 12 comprend, plus particulièrement est constitué, d'une première partie 14 et une deuxième partie 16.

La première partie 14 correspond à une partie inférieure, et la deuxième partie 16 correspond à une partie supérieure.

La partie inférieure 14 est ici formée de deux portions 18, 20 prévues pour s'étendre autour des cuisses d'un utilisateur. La partie supérieure 16 est prévue pour s'étendre autour du bassin d'un utilisateur et de relier les deux portions 18, 20 de la partie inférieure 14.

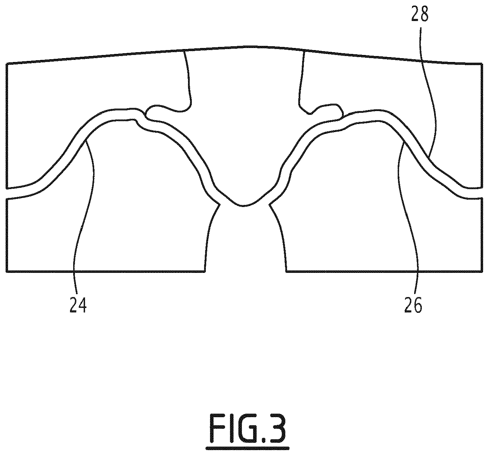

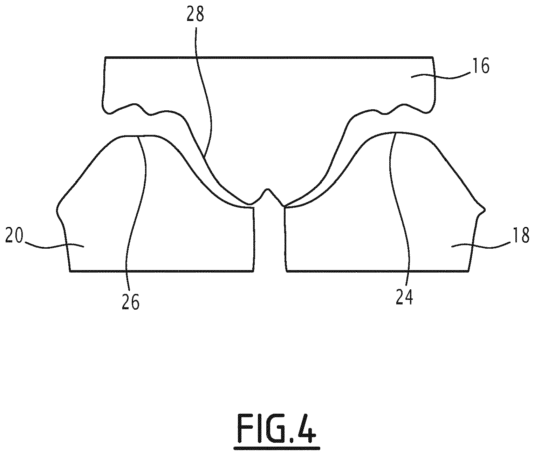

La partie inférieure 14 et la partie supérieure 16 sont chacune apte à être découpée dans un patron plat visible sur les figures 3 et 4.

Plus particulièrement, chaque portion 18, 20 de la partie inférieure 14 est réalisée à partir d'une pièce textile plate, deux bords de la pièce plate susceptible d'être reliés, par exemple cousus, ensemble de manière à former une forme globalement tubulaire.

La partie supérieure 16 est réalisée à partir d'une pièce textile plate, deux bords 22 de la pièce susceptible d'être reliés, par exemple cousus, ensemble, plus particulièrement à l'avant du vêtement 10.

La partie inférieure 14 et la partie supérieure 16 comprennent chacun un bord de connexion.

Plus particulièrement, chaque portion 18, 20 de la partie inférieure 14 présente un bord de connexion 24, 26, lesdits bords de connexion 24, 26 formant ensemble le bord de connexion de la partie inférieure 14.

Le bord de connexion 24, 26 de la partie inférieure 14 est relié au bord de connexion 28 de la partie supérieure 16.

Plus particulièrement, le bord de connexion 24, 26 de la partie inférieure 14 et le bord de connexion 28 de la partie supérieure 16 sont cousus l'un à l'autre au niveau de coutures 29.

Le bord de connexion 24, 26 de la partie inférieure 14 et le bord de connexion 28 de la partie supérieure 16 ne sont pas complémentaires l'un de l'autre.

Le lien entre la partie inférieure 14 et la partie supérieure 16 au niveau des bords de connexion 24, 26, 28 forme alors une pince du vêtement.

Cela permet notamment de suivre la morphologie du corps.

La pince est prévue pour s'étendre au moins contre l'aine et/ou le pli fessier d'un utilisateur, plus particulièrement contre l'aine et le pli fessier d'un utilisateur.

Le patron respectif des parties inférieure et supérieure est prévu pour que les bords de connexion 24, 26, 28 s'étendent au niveau de l'aine et du pli fessier d'un utilisateur.

Tout autre mode de réalisation du corps principal 12 permettant d'être adapté à la morphologie d'un utilisateur est envisageable.

Les pinces sont, par exemple, situées à un autre emplacement du corps principal.

Alternativement, le patron est découpé d'une pièce de sorte que le corps principal 12 soit ajusté sur l'utilisateur. Par exemple, pour un modèle homme, un patron de coque prévu pour englober les parties génitales est prévu.

Le vêtement 10 présente au moins une première zone ciblée 30, plus particulièrement une unique première zone ciblée.

Dans l'exemple représenté, sur l'ensemble de la première zone ciblée 30, le vêtement comprend au moins une couche de textile de renforcement 32.

Plus particulièrement, ici, le corps principal 12, plus particulièrement sa face externe, est revêtu d'une couche de textile de renforcement 32.

Le textile de renforcement 32 est configuré pour ne pas s'étendre directement contre un utilisateur.

Dans un mode de réalisation non représenté, le textile de renforcement 32 ne s'étend pas sur l'ensemble de la première zone ciblée 30, mais uniquement sur une portion de la première zone ciblée.

Le textile de renforcement 32 est élastique et possède un allongement maximal supérieur à 50%, c'est-à-dire qu'il est susceptible d'être allongé d'au moins 50% sans subir de dommages.

L'allongement maximal d'un matériau est ici mesuré par étirement d'une bande présentant une largeur donnée, ici 5 centimètres, un nombre donné de cycles d'étirage, ici égal à 5, selon une direction d'intérêt perpendiculaire à la mesure de la largeur. Pour chaque cycle, la bande est étirée par une force augmentant progressivement jusqu'à une force prédéterminée, par exemple égale à 6N par centimètre de largeur de la bande, ici donc jusqu'à 30N. L'allongement maximal est ici l'allongement à la force prédéterminée lors du dernier cycle d'étirage, soit ici l'allongement à 30N au cinquième cycle d'étirage.

L'allongement maximal du textile de renforcement est strictement supérieur à l'allongement de la première zone ciblée 30 lorsque le vêtement est porté par un utilisateur.

Plus le module du textile de renforcement 32 est important, plus son action de renfort compressif est importante.

Le vêtement présente un module supérieur au module du textile de compression dans la première zone ciblée 30.

Le textile de renforcement 32 présente selon un mode de réalisation un module supérieur à celui du textile de compression.

Selon un autre mode de réalisation de l'invention, le module du textile de renfort est inférieur ou égal au module du textile de compression.

Le textile de renforcement 32 est prévu pour s'étendre au moins contre l'aine et/ou le fessier d'un utilisateur.

Plus particulièrement, la première zone ciblée 30 s'étend sur des parties du vêtement destinées à s'étendre au moins en regard de l'ensemble du fessier, du pli fessier, de la zone pubienne et des creux de l'aine.

Dans l'exemple représenté, la première zone ciblée 30 s'étend uniquement en regard de l'ensemble du fessier, du pli fessier, de la zone pubienne et des creux de l'aine.

Dans un mode de réalisation alternatif, la première zone ciblée 30 s'étend en outre sur l'intérieur des cuisses.

La première zone ciblée 30 est ici formée d'une unique zone continue.

Le textile de renforcement 32 est cousu sur le ou au corps principal 12, par exemple avec des coutures de recouvrement 34 s'étendant sur l'ensemble de la périphérie de la première zone ciblée.

Les coutures 34 sont telles qu'elles ne limitent pas l'étirage du corps principal 12 et de la première zone ciblée 30, sur la plage d'utilisation prévue du vêtement.

Le textile de renforcement 32 comprend, par exemple, plus particulièrement est constitué, de polyamide et d'élasthanne. Le textile de renforcement 32 est ici constitué de 40% à 50%, plus particulièrement 48%, d'élasthanne et de 50% à 60%, plus particulièrement 52%, de polyamide.

Le textile de renforcement 32 est ici un tissu bi-élastique, c'est-à-dire élastique selon deux directions d'extension du tissu non parallèle, ici la direction d'extensibilité et la direction perpendiculaire à la direction d'extensibilité.

Alternativement, le textile de renforcement 32 est identique au textile de compression.

Le vêtement 10 présente au moins une deuxième zone ciblée.

Dans l'exemple représenté, le vêtement 10 présente deux deuxièmes zones ciblées 36, 38.

Dans, plus particulièrement sur l'ensemble de, la ou chaque deuxième zone ciblée 36, 38, le vêtement comprend une couche de textile de contention 40.

Dans l'exemple représenté, le corps principal 12, plus particulièrement sa face interne, est revêtu d'une couche de textile de contention 40.

Le textile de contention est apte à rigidifier la zone qu'il couvre de manière à contenir le gonflement de l'oedème.

Le textile de contention présente une rigidité le long d'au moins une direction d'intérêt sur sa plage d'étirage d'utilisation du vêtement. L'au moins une direction d'intérêt est ici la direction circonférentielle.

D'après la norme « NF S 97-115 Décembre 2011 - Bandes textiles de compression et de contention médicales », la rigidité R est la « caractéristique d'une variation de pression obtenue par la relation de proportionnalité entre la force F appliquée en un point et la modification d'allongement résultante en ce point », et se calcule de la manière suivante :

$$R = \frac{F2-F1}{A2-A1}$$ , exprimée en N/(m*mm), où

A1 et A2 sont des allongements de la matière dont la rigidité est définie, exprimé en millimètres (mm), et

F1 et F2 sont les forces de traction pour étirer la matière respectivement aux allongements A1 et A2 exprimées en Newtons par mètre (N/m), correspondant respectivement aux allongements A1 et A2.

Les allongements A1 et A2 sont ici choisis de sorte à être situés dans la zone d'utilisation préconisée du vêtement.

Le textile de contention a par exemple une rigidité selon la direction d'intérêt supérieure ou égale à 30 N/(m*mm), plus particulièrement est comprise entre 30 et 75 N/(m*mm), sur la plage correspondant à une utilisation normale du vêtement.

La rigidité du textile de contention dans la direction perpendiculaire à la direction d'intérêt est strictement inférieure à la rigidité du textile de contention selon la direction d'intérêt, sur la plage correspondant à une utilisation normale du vêtement. Cela permet notamment une augmentation du confort de l'utilisateur portant le vêtement.

La rigidité du textile de contention selon la direction d'intérêt est strictement supérieure à la rigidité du corps principal selon la direction d'intérêt, plus particulièrement d'au moins 100%, sur la plage correspondant à une utilisation normale du vêtement.

La rigidité du textile de contention selon la direction d'intérêt est strictement supérieure à la rigidité du vêtement dans la première zone ciblée selon la direction d'intérêt, plus particulièrement d'au moins 100%, sur la plage correspondant à une utilisation normale du vêtement.

Le textile de contention a par exemple une rigidité selon la direction d'intérêt strictement supérieure à la rigidité du reste du vêtement, sur la plage correspondant à une utilisation normale du vêtement.

La ou l'une des deuxièmes zones ciblées, dite deuxième zone ciblée inférieure 36, est prévue pour s'étendre contre l'intérieur des cuisses. La deuxième zone ciblée inférieure 36 s'étend ici uniquement contre l'intérieur des cuisses et au niveau de l'entrejambe.

Le textile de contention de la deuxième zone ciblée inférieure 36 est ici solidaire du corps principal 12, par exemple par couture, au moins sur l'ensemble de la périphérie de la deuxième zone ciblée inférieure 36.

L'autre des deuxièmes zones ciblées, dite deuxième zone ciblée supérieure 38, est prévue pour s'étendre contre la zone pubienne d'un utilisateur.

Dans un mode de réalisation, la deuxième zone ciblée supérieure 38 s'étend uniquement contre la zone pubienne.

Alternativement, la deuxième zone ciblée supérieure 38 s'étend uniquement contre la zone pubienne et les zones inguinales.

La deuxième zone ciblée supérieure 38 est ici incluse dans la première zone ciblée 30, de sorte que dans la deuxième zone ciblée supérieure 38, le vêtement comprend une couche de textile de renforcement et une couche de textile de contention.

Le textile de contention de la deuxième zone ciblée supérieure 38 est ici solidaire du corps principal 12, par exemple par couture, sur une portion uniquement de la périphérie de la deuxième zone ciblée inférieure 38, le textile de contention formant avec le corps principal 12 une poche 42.

Un élément de capitonnage est ici inséré dans la poche 42. L'élément de capitonnage est amovible de la poche 42.

La poche apporte avantageusement de la contention de part la nature du textile et sa rigidité au port, ainsi que du maintien via l'élément de capitonnage, améliorant le confort de vie d'un utilisateur lors du port du vêtement.

L'élément de capitonnage est apte à évacuer des écoulements de lymphe depuis la peau, par exemple par captation de liquide, notamment par effet de capillarité.

L'élément de capitonnage est, par exemple, réalisée en polyamide. Cela permet notamment un séchage rapide de l'élément de capitonnage.

L'élément de capitonnage est, par exemple, une bande de Mobiderm © de Thuasne © à petits plots recouverte sur ses deux faces par un textile en polyamide.

La poche permet au dispositif de capitonnage de rester correctement positionné au cours de l'utilisation du produit par l'utilisateur, améliorant la qualité de vie de l'utilisateur lors de son usage.

La conception du vêtement permet notamment d'appliquer des efforts différemment pour aider à drainer le membre oedématié en le contenant et en étant apte à s'adapter à la morphologie de la zone à traiter et/ou à appliquer une pression à des endroits précis où celle-ci doit être plus importante pour drainer efficacement le membre.

La première zone ciblée permet notamment d'apporter plus de maintien par une rigidité plus importante qu'en dehors des zones ciblées.

La deuxième zone ciblée permet d'appliquer une force importante dès que le volume du membre augmente d'une valeur minimale, par exemple, en cas de gonflement du lymphoedème, de manière à drainer efficacement un lymphoedème formant un gonflement.

Dans un mode de réalisation alternatif, le corps principal 12 s'étend sur l'ensemble de la surface du vêtement 10 à l'exception d'au moins une zone ouverte, plus particulièrement à l'exception uniquement de la ou des deuxièmes zones ciblées. Seules les différences avec le mode de réalisation décrit précédemment sont décrites ensuite.

Le textile de contention de la ou de chacune des deuxièmes zones ciblées 36, 38 est ici connecté au corps principal 12, par exemple par couture, sur la périphérie de la deuxième zone ciblée correspondante 36, 38.

Plus particulièrement, le textile de contention de la deuxième zone ciblée inférieure 36 est connecté au corps principal 12 sur l'ensemble de la périphérie de la deuxième zone ciblée inférieure 36 en contact avec le corps principal 12.

Le textile de contention de la deuxième zone ciblée supérieure 38 est ici solidaire du corps principal 12, par exemple par couture, sur une portion uniquement de la périphérie de la deuxième zone ciblée inférieure 38.

Dans la partie de la première zone ciblée faisant face à la deuxième zone ciblée, le textile de renforcement ne revêt alors pas le corps principal. Le textile de renforcement s'étend directement contre le textile de contention.

Le textile de contention s'étend sur la face interne du textile de renforcement dans la partie de la première zone ciblée faisant face à la deuxième zone ciblée.

Le textile de contention forme avec le textile de renforcement une poche similaire à celle décrite précédemment entre le textile de contention et le corps principal.

Le textile de renforcement est configuré pour ne pas s'étendre directement contre un utilisateur, sur l'ensemble de sa surface.

Exemple 1

Le vêtement est, par exemple, conçu avec un modèle homme et un modèle femme de manière à s'adapter à la morphologie spécifique et différentes tailles pour chaque modèle.

Un tableau de taillage pour le modèle homme et le modèle femme est défini selon la circonférence du bassin de manière à ce que le vêtement soit adapté à la morphologie de l'utilisateur :

| Taille | 1 | 2 | 3 | 4 | 5 | 6 |
|---|---|---|---|---|---|---|
| Circonférence du bassin (cm) | 88-94 | 95-101 | 102-108 | 109-115 | 116-122 | 123-129 |
| Circonférence moyenne du bassin (cm) | 91 | 98 | 105 | 112 | 119 | 126 |

Pour chaque modèle et chaque taille, la force nécessaire pour étirer le vêtement à la circonférence moyenne du bassin est calculée grâce à la loi de Laplace relative à la pression de compression qui stipule que la force à appliquer en N par centimètre de largeur, la largeur étant mesurée perpendiculairement à la direction circonférentielle, est égale à la pression appliquée en hectopascal multipliée par le rayon sur lequel est appliquée la force divisé par 100.

Pour une circonférence moyenne du bassin égale à 112 cm et une pression visée de 15mmHg, on obtient le calcul suivant :

$$F\,(N.cm^{-1}) = R(cm) \times \frac{P(hPa)}{100} = \frac{112(cm)}{2\pi} \times \frac{(15(mmHg) \times 1{,}33322)}{100} = 3{,}57\,N/cm$$

Pour chaque matière parmi le textile de compression, le textile de renforcement et le textile de contention, une bande est étirée en appliquant une force augmentant progressivement, tel que décrit précédemment, par exemple jusqu'à une valeur de force d'étirage égale à 6 N par centimètres de largeur de la bande, sur au moins cinq cycles.

Pour chaque matière, on relève sur la courbe correspondant à l'étirage de la matière par augmentation de la force au premier cycle, le pourcentage de la matière pour avoir cette force.

Ici pour une bande de cinq centimètres de largeur, on relève pour chaque matière, l'étirage de la matière correspondant à une force de 17,83 N.

Les patrons pour le corps principal, le textile de renforcement et le textile de contention sont alors conçus de manière à ce que chaque matière soit étirée de ce pourcentage lorsque le vêtement est porté par une personne présentant une circonférence de bassin égale à la circonférence moyenne de bassin, dans cet exemple égale à la circonférence moyenne en taille 4.

Ainsi, un utilisateur présentant une circonférence de bassin égale à la circonférence moyenne, ici dans l'exemple de taille 4, et portant un vêtement selon la taille prescrite se voit appliquer une force de 15 mmHg.

Exemple 2

Le vêtement est conçu tel que décrit dans le premier exemple.

L'allongement du vêtement nécessaire pour atteindre les extrémités de chaque gamme correspondant à une circonférence de bassin est calculé en connaissant le patron du vêtement non étiré.

La force nécessaire par centimètre de largeur pour atteindre un tel allongement est alors déduite des courbes correspondant à l'étirage de chaque matière par augmentation de la force au premier cycle, telles que décrites précédemment.

La loi de Laplace permet alors de calculer la pression appliquée par le vêtement pour chaque extrémité de chaque gamme correspondant à une circonférence de bassin.

$$P(mmHg) = \frac{F\,(N.cm^{-1}) \times 100}{R} \times \frac{1}{1,33322}$$

Ainsi, les vêtements appliquent des pressions moyennes comprises entre 12 mmHg et 18 mmHg sur l'utilisateur ce qui correspond à une gamme de valeurs classique dans le traitement du lymphoedème pour que le vêtement ait une action mais que la pression soit tolérée par un utilisateur.

Exemple 3

Un vêtement a été enfilé par des utilisateurs correspondant à leur sexe et à leur mesure de circonférence de bassin, des capteurs de pression commercialisés sous l'appellation PicoPress ® de MICROLAB étant disposés entre le vêtement et l'utilisateur en différentes zones : pubis, aine, côté de chaque jambe et fessier.

Les vêtements appliquent des pressions en différents emplacements comprises entre 7 mmHg et 21 mmHg sur l'utilisateur ce qui correspond à une gamme de valeurs classique dans le traitement du lymphoedème pour que le vêtement ait une action mais que la pression soit tolérée par un utilisateur.

L'allongement du vêtement nécessaire pour atteindre les extrémités de chaque gamme correspondant à une circonférence de bassin est calculé en connaissant le patron du vêtement non étiré.

La force nécessaire par centimètre de largeur pour atteindre un tel allongement est alors déduite des courbes correspondant à l'étirage de chaque matière par augmentation de la force au premier cycle, telles que décrites précédemment.

La loi de Laplace permet alors de calculer la pression appliquée par le vêtement pour chaque extrémité de chaque gamme correspondant à une circonférence de bassin.

$$P(mmHg) = \frac{F\,(N.cm^{-1}) \times 100}{R} \times \frac{1}{1,33322}$$

Ainsi, les vêtements appliquent des pressions moyennes comprises entre 12 mmHg et 18 mmHg sur l'utilisateur ce qui correspond à une gamme de valeurs classique dans le traitement du lymphoedème pour que le vêtement ait une action mais que la pression soit tolérée par un utilisateur.

Exemple 3

Un vêtement a été enfilé par des utilisateurs correspondant à leur sexe et à leur mesure de circonférence de bassin, des capteurs de pression commercialisés sous l'appellation PicoPress ® de MICROLAB étant disposés entre le vêtement et l'utilisateur en différentes zones : pubis, aine, côté de chaque jambe et fessier.

Les vêtements appliquent des pressions en différents emplacements comprises entre 7 mmHg et 21 mmHg sur l'utilisateur ce qui correspond à une gamme de valeurs classique dans le traitement du lymphoedème pour que le vêtement ait une action mais que la pression soit tolérée par un utilisateur.

**Revendications**

1. Vêtement (10) de compression et/ou de contention pour le traitement du lymphoedème, le vêtement (10) comprenant un corps principal (12), le corps principal (12) étant réalisé dans un textile de compression, le textile de compression présentant une élasticité strictement supérieure à 70% dans au moins une direction d'extensibilité, le textile de compression présentant un module selon la norme ISO NF EN 14704-1 de juin 2005 sur la détermination de l'élasticité des étoffes supérieure ou égale à 3,5 N à un allongement de 40% sur la cinquième courbe de charge selon la direction d'extensibilité,

   le vêtement (10) présentant au moins une première zone ciblée (30), dans laquelle le vêtement comprend au moins une couche de textile de renforcement (32) dans la première zone ciblée (30), le vêtement (10) présentant dans la première zone ciblée (30) un module supérieur au module du textile de compression,
   le vêtement (10) présentant au moins une deuxième zone ciblée (36, 38) dans laquelle le vêtement (10) comprend au moins une couche de textile de contention (40), le textile de contention (40) présentant une rigidité le long d'au moins une direction d'intérêt supérieure ou égale à 30 N/(m*mm) sur toute plage d'utilisation normale du vêtement (10),
   la rigidité du textile de contention (40) selon la direction d'intérêt étant strictement supérieure à la rigidité du corps principal (12) selon la direction d'intérêt sur une plage d'utilisation normale du vêtement (10),
   la rigidité du textile de contention (40) selon la direction d'intérêt étant strictement supérieure à la rigidité du vêtement (10) dans la première zone ciblée (30) selon la direction d'intérêt, plus particulièrement d'au moins 100%.

2. Vêtement de compression selon la revendication 1, dans lequel la direction d'extensibilité du textile de compression et/ou la direction d'intérêt correspondent à une direction circonférentielle du vêtement (10).

3. Vêtement de compression selon la revendication 1 ou 2, dans lequel le textile de compression est prévu pour que le textile de compression requiert une force d'étirage selon la direction d'extensibilité supérieure ou égale à 7N pour obtenir un allongement de 40% lors d'un premier cycle d'étirage.

4. Vêtement de compression selon l'une quelconque des revendications 1 à 3, dans lequel le textile de contention (40) est prévu pour s'étendre au moins contre la zone pubienne d'un utilisateur et/ou l'intérieur des cuisses d'un utilisateur.

5. Vêtement de compression selon l'une quelconque des revendications 1 à 4, dans lequel le textile de renforcement (32) est prévu pour s'étendre au moins contre l'aine, le pli fessier, la zone pubienne et/ou le fessier d'un utilisateur.

6. Vêtement de compression selon l'une quelconque des revendications 1 à 5, dans lequel le corps principal (12) s'étend sur l'ensemble du vêtement (10) à l'exception de la ou de chacune des ou d'au moins une des deuxièmes zones ciblées (36, 38).

7. Vêtement de compression selon l'une quelconque des revendications 1 à 6, dans lequel le corps principal (12) comprend au moins une première partie (14) et une deuxième partie (16), la première partie (14) et la deuxième partie (16) étant chacune apte à être découpée dans un patron plat, la première partie (14) et la deuxième partie (16) comprenant chacune un bord de connexion (24, 26), le bord de connexion (24) de la première partie (14) et le bord de connexion (26) de la deuxième partie (16) n'étant pas complémentaire l'un de l'autre, le bord de connexion (24) de la première partie (14) étant relié au bord de connexion (26) de la deuxième partie (16) par une pince.

8. Vêtement de compression selon la revendication 6 ou 7, dans lequel la pince est prévue pour s'étendre au moins contre l'aine et/ou le pli fessier d'un utilisateur.

9. Vêtement de compression selon l'une quelconque des revendications 1 à 8, dans lequel le textile de compression et/ou le textile de renforcement (32) comprend, plus particulièrement est constitué, de polyamide et d'élasthanne.

10. Vêtement de compression selon l'une quelconque des revendications 1 à 9, présentant au moins une poche (42) apte à recevoir de manière amovible au moins un élément de capitonnage.

**EP 4 061 168 B1**

**Patentansprüche**

1.  Kompressions- und/oder Stützbekleidung (10) zur Behandlung von Lymphödemen, wobei die Bekleidung (10) einen Hauptkörper (12) umfasst, wobei der Hauptkörper (12) aus einem Kompressionstextil hergestellt ist, wobei das Kompressionstextil eine Elastizität strikt über 70 % in mindestens einer Dehnbarkeitsrichtung aufweist, wobei das Kompressionstextil ein Modul gemäß der Norm ISO NF EN 14704-1 von Juni 2005 zur Bestimmung der Elastizität von Stoffen von über oder gleich 3,5 N bei einer Längung von 40 % auf der fünften Belastungskurve entlang der Dehnbarkeitsrichtung aufweist,

    wobei die Bekleidung (10) mindestens einen ersten Zielbereich (30) aufweist, wobei die Bekleidung mindestens einen ersten Zielbereich (30) aufweist, in dem die Bekleidung mindestens eine Schicht aus Verstärkungstextil (32) im ersten Zielbereich (30) umfasst, wobei die Bekleidung (10) im ersten Zielbereich (30) ein Modul über dem Modul des Kompressionstextils aufweist,
    wobei die Bekleidung (10) mindestens einen zweiten Zielbereich (36, 38) aufweist, in dem die Bekleidung (10) mindestens eine Schicht eines Stütztextils (40) umfasst, wobei das Stütztextil (40) eine Steifigkeit entlang mindestens einer interessierenden Richtung von über oder gleich 30 N/(m*mm) über den gesamten normalen Verwendungsbereich der Bekleidung (10) aufweist,
    wobei die Steifigkeit des Stütztextils (40) gemäß der interessierenden Richtung strikt über der Steifigkeit des Hauptkörpers (12) gemäß der interessierenden Richtung auf einem normalen Verwendungsbereich der Bekleidung (10) liegt,
    wobei die Steifigkeit des Stütztextils (40) gemäß der interessierenden Richtung strikt über der Steifigkeit der Bekleidung (10) im ersten Zielbereich (30) gemäß der interessierenden Richtung liegt, insbesondere um mindestens 100 %.

2.  Kompressionsbekleidung nach Anspruch 1, wobei die Dehnbarkeitsrichtung des Kompressionstextils und/oder die interessierende Richtung einer Umfangsrichtung der Bekleidung (10) entsprechen.

3.  Kompressionsbekleidung nach Anspruch 1 oder 2, wobei das Kompressionstextil vorgesehen ist, dass das Kompressionstextil eine Streckkraft gemäß der Dehnbarkeitsrichtung von über oder gleich 7 N benötigt, um in einem ersten Streckzyklus eine Längung von 40 % zu erzielen.

4.  Kompressionsbekleidung nach einem der Ansprüche 1 bis 3, wobei das Stütztextil (40) vorgesehen ist, sich zumindest gegen den Schambereich eines Benutzers und/oder die Innenseite der Oberschenkel eines Benutzers zu erstrecken.

5.  Kompressionsbekleidung nach einem der Ansprüche 1 bis 4, wobei das Verstärkungstextil (32) vorgesehen ist, sich zumindest gegen die Leiste, die Gesäßfalte, den Schambereich und/oder das Gesäß eines Benutzers zu erstrecken.

6.  Kompressionsbekleidung nach einem der Ansprüche 1 bis 5, wobei sich der Hauptkörper (12) über die gesamte Bekleidung (10) mit Ausnahme des oder jedes oder mindestens eines der zweiten Zielbereiche (36, 38) erstreckt.

7.  Kompressionsbekleidung nach einem der Ansprüche 1 bis 6, wobei der Hauptkörper (12) mindestens einen ersten Teil (14) und einen zweiten Teil (16) umfasst, wobei der erste Teil (14) und der zweite Teil (16) jeweils imstande sind, aus einer flachen Schablone ausgeschnitten zu werden, wobei der erste Teil (14) und der zweite Teil (16) jeweils eine Verbindungskante (24, 26) umfassen, wobei die Verbindungskante (24) des ersten Teils (14) und die Verbindungskante (26) des zweiten Teils (16) nicht komplementär zueinander sind, wobei die Verbindungskante (24) des ersten Teils (14) mit der Verbindungskante (26) des zweiten Teils (16) durch eine Klammer verbunden ist.

8.  Kompressionsbekleidung nach Anspruch 6 oder 7, wobei die Klammer vorgesehen ist, sich zumindest gegen die Leiste und/oder die Gesäßfalte eines Benutzers zu erstrecken.

9.  Kompressionsbekleidung nach einem der Ansprüche 1 bis 8, wobei das Kompressions- und/oder Verstärkungstextil (32) Polyamid und Elasthan umfasst, insbesondere daraus besteht.

10. Kompressionsbekleidung nach einem der Ansprüche 1 bis 9, die mindestens einer Tasche (42) aufweist, die geeignet ist, mindestens ein Polsterelement lösbar aufzunehmen.

9

**Claims**

1. A compression and/or contention garment (10) for lymphoedema treatment, the garment (10) comprising a main body (12), the main body (12) being made of a compression textile, the compression textile having an elasticity strictly greater than 70% in at least one direction of extensibility, the compression textile having a module in accordance with the ISO standard NF EN 14704-1 of June 2005 on the determination of the elasticity of fabrics that is greater than or equal to 3.5 N at an elongation of 40% on the fifth load curve in the direction of extensibility,

   the garment (10) having at least a first targeted area (30), in which the garment comprises at least one layer of reinforcement textile (32) in the first targeted area (30), the garment (10) having a module in the first targeted area (30) that is greater than the module of the compression textile,
   the garment (10) having at least a second targeted area (36, 38) in which the garment (10) comprises at least one layer of contention textile (40), the contention textile (40) having a stiffness in at least one direction of interest greater than or equal to 30 N/(m*mm) over any normal range of use of the garment (10),
   the stiffness of the contention textile (40) in the direction of interest being strictly greater than the stiffness of the main body (12) in the direction of interest over a normal range of use of the garment (10),
   the stiffness of the contention textile (40) in the direction of interest being strictly greater than the stiffness of the garment (10) in the first targeted area (30) in the direction of interest, more particularly by at least 100%.

2. The compression garment according to claim 1, in which the direction of extensibility of the compression textile and/or the direction of interest correspond to a circumferential direction of the garment (10).

3. The compression garment according to claim 1 or 2, in which the compression textile is provided such that the compression textile requires a stretching force, in the direction of extensibility, greater than or equal to 7N, to achieve 40% elongation in a first stretching cycle.

4. The compression garment according to any one of claims 1 to 3, in which the contention textile (40) is intended to extend at least against a user's pubic area and/or a user's inner thighs.

5. The compression garment according to any one of claims 1 to 4, in which the reinforcement textile (32) is intended to extend at least against a user's groin, buttock crease, pubic area and/or buttocks.

6. The compression garment according to any one of claims 1 to 5, in which the main body (12) extends over the entire garment (10) except for the or each or at least one of the second targeted areas (36, 38).

7. The compression garment according to any one of claims 1 to 6, in which the main body (12) comprises at least a first part (14) and a second part (16), the first part (14) and the second part (16) each being capable of being cut from a flat pattern, the first part (14) and the second part (16) each comprising a connecting edge (24, 26), the connecting edge (24) of the first part (14) and the connecting edge (26) of the second part (16) not complementing each other, the connecting edge (24) of the first part (14) being connected to the connecting edge (26) of the second part (16) by a dart.

8. The compression garment according to claim 6 or 7, in which the dart is intended to extend at least against a user's groin and/or buttock crease.

9. The compression garment according to any one of claims 1 to 8, in which the compression textile and/or reinforcement textile (32) comprises, more particularly consists of polyamide and elastane.

10. The compression garment according to any one of claims 1 to 9, having at least one pocket (42) adapted to removably receive at least one padding element.

## FIG.1

## FIG.2

## FIG.3

FIG.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2018303179 A1
- EP 2449901 A2
- US 2015173428 A1
- WO 2011124849 A1
- FR 3027195 A1